(19) Europäisches Patentamt
European Patent Office
Office européen des brevets

(11) Veröffentlichungsnummer : **0 035 027**
**B1**

(12) # EUROPÄISCHE PATENTSCHRIFT

(45) Veröffentlichungstag der Patentschrift : **15.05.85**

(51) Int. Cl.⁴ : **C 08 G 65/32**

(21) Anmeldenummer : **80901772.6**

(22) Anmeldetag : **28.08.80**

(86) Internationale Anmeldenummer : **PCT/EP 80/00085**

(87) Internationale Veröffentlichungsnummer : **WO/8100571 (05.03.81 Gazette 81/06)**

(54) **NEUE SÄUREN DES PHOSPHORS UND DEREN DERIVATE.**

(30) Priorität : **30.08.79 DE 2935134**
**24.10.79 DE 2943016**

(43) Veröffentlichungstag der Anmeldung : **09.09.81 Patentblatt 81/36**

(45) Bekanntmachung des Hinweises auf die Patenterteilung : **15.05.85 Patentblatt 85/20**

(84) Benannte Vertragsstaaten : **AT CH DE FR GB LI LU NL SE**

(56) Entgegenhaltungen :
**FR-A- 2 314 243**
**US-A- 4 154 674**
**Chemical Abstracts, Band 81, Nr. 22, 02. Dezember 1974 (Columbus, Ohio, US), siehe Seite 13, Zusammenfassung 136740w JP, A, 7451400, 18, Mai 1974, Toyobo Co., Ltd.**
**Derwent, Band U, Nr. 37, 12. September 1973, siehe Zusammenfassung 54311U JP, B, 7329141, 25. Januar 1969, Kamata K., et al.**
**Die Akte enthält technische Angaben, die nach dem Eingang der Anmeldung eingereicht wurden und die nicht in dieser Patentschrift enthalten sind.**

(73) Patentinhaber : **Gesellschaft für Biotechnologische Forschung mbH (GBF)**
**Mascheroder Weg 1**
**D-3300 Braunschweig-Stöckheim (DE)**

(72) Erfinder : **Morr, Michael, Dr. Dipl.-Chem.**
**Hohe Wiese 43**
**D-3300 Braunschweig (DE)**
Erfinder : **Kula, Maria-Regina, Dr. Dipl.-Chem.**
**Forstweg 15**
**D-3340 Wolfenbüttel (DE)**

(74) Vertreter : **Boeters, Hans Dietrich, Dr. et al**
**Boeters, Bauer & Partner Thomas-Wimmer-Ring 14**
**D-8000 München 22 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 035 027 B1

**Beschreibung**

Die Erfindung betrifft Verbindungen der allgemeinen Formel

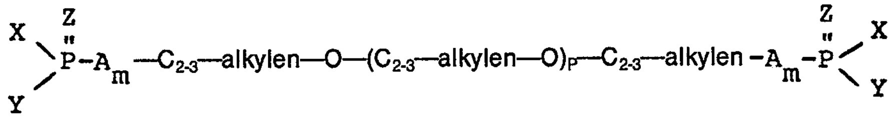

mit p = 1 bis 800 und

(a1) m = 1,
A = O—Atom,
X = Cl oder OH und Y = X oder —O—$C_{1-5}$—alkyl,
Z = O— oder S—Atom ; wobei für X = OH, Y = —O—$C_{1-5}$—alkyl und
Z = O—Atom p kleiner oder gleich 100 ausgenommen ist, oder

(a2) m = 1,
A = O—Atom,

X = —O—$C_{1-5}$—alkyl, —O—$C_{1-5}$—alkylen— 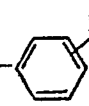, 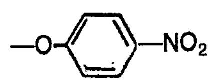 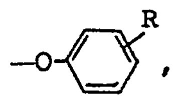,

—NH—$C_{1-5}$—alkyl, —N($C_{1-5}$—alkyl)$_2$, —NH—$C_{1-5}$—alkylen— 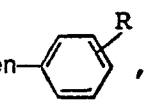, —N($C_{1-5}$—alkylen— 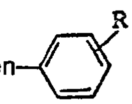 )$_2$

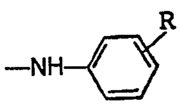 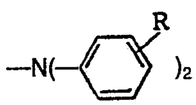

Y = X oder —O—$C_{1-5}$—alkyl,
R = OH, $C_{1-5}$—alkyl, —O—$C_{1-5}$—alkyl oder fehlt,
Z = O—Atom ; wobei für X = —N($C_{1-5}$—alkyl)$_2$ oder —O—$C_{1-5}$—alkyl p kleiner oder gleich 7 ausgenommen ist, oder

(a3) m = 1,
A = O—Atom,
X = OH,
Y = —S—$C_{1-5}$—alkyl, —S—amino—$C_{2-5}$—alkyl, S—carboxy—$C_{1-5}$—alkyl,
Z = O—Atom ; oder

(b) m = 1,
A = S—Atom,
X = Y = OH,
Z = O—Atom ; oder

(c) m = 0,

X = Y = OH, —O—$C_{1-5}$—alkyl, —O—$C_{1-5}$—alkylen— 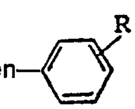, 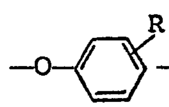 —O—Si($C_{1-5}$—alkyl)$_3$,

R = OH, $C_{1-5}$—Alkyl, —O—$C_{1-5}$—alkyl oder fehlt,
Z = O—Atom,

und deren Derivate, bei denen einer der beiden 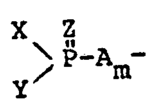 -Gruppen durch eine $C_{1-5}$—Alkoxygruppe ersetzt ist,
und die Alkalimetallderivate und Salze mit Ammoniak und Aminen der Säuren des Phosphors und der Carbonsäuren.

Die Polyätherkette der vorstehenden Verbindungen baut sich aus Äthylenglykol-, Propylenglykol- oder i-Propylenglykoleinheiten oder mehreren dieser Einheiten auf ; p kann dabei einen Wert von 3 bis 250 und vorzugsweise 30 bis 160 besitzen.

Beispiele für Alkoxyreste sind der Methoxy-, Äthoxy- und Propoxyrest. Beispiele für Alkylamino- und Dialkylaminoreste sind der Methylamino-, Äthylamino-, Propylamino- bzw. Dimethylamino-, Diäthylamino- und Dipropylaminorest. Beispiele für Alkylthioreste sind Alkylthioreste mit 1 bis 3 Kohlenstoffatomen.

Beispiele für Aminoalkylthioreste sind Aminoalkylthioreste mit 2 bis 3 Kohlenstoffatomen. Beispiele für Carboxyalkylthioreste sind Carboxyalkylthioreste mit 1 bis 3 Kohlenstoffatomen in der Alkylengruppe. Ein Beispiel für einen Trialkylsilyloxyrest ist der Trimethylsilyloxyrest.

Bei den Kernsubstituenten der kernsubstituierten Phenylalkoxy-, Phenylalkylamino- und Bisphenylalkylaminoreste kann es sich um einen Methyl-, Äthyl-, Propyl-, Methoxy-, Äthoxy- und/oder Propoxyrest handeln.

Bei den Salzen handelt es sich vorzugsweise um Salze der Säuren des Phosphors, vorzugsweise um Ammonium-, Lithium-, Natrium- oder Kaliumsalze.

Zur Herstellung sämtlicher Verbindungen kann man von Polyäthylenglykol, Polypropylenglykol, Polyi-propylenglykol oder ihren Mischpolymeren ausgehen. Nachstehend werden Herstellungsmethoden beschrieben.

I) Herstellung von Verbindungen der allgemeinen Formel mit m = 1 ; A = Sauerstoffatom ; X = Chloratom oder Hydroxylrest ; Y = X oder Alkoxyrest mit 1 bis 5 Kohlenstoffatomen ; Z = Sauerstoffatom

Zur Herstellung der Phosphorsäurehalogenide kann man ein Polyalkylenglykol mit Phosphoroxychlorid oder Pyrophosphorylchlorid ohne oder mit Lösungsmittel, wie Phosphorsäuretrialkylester, z. B. Triäthylphosphat, oder Dichlormethan, in Gegenwart eines Säurefängers umsetzen, z. B. einer tertiären Base, wie Triäthylamin oder Pyridin, oder eines Molekularsiebes (mit z. B. 0,4 nm). Die erhaltenen Phosphorsäurehalogenide kann man mit Wasser oder zuerst mit der halben stöchiometrischen Menge eines $C_{1-5}$—Alkohols und danach mit Wasser zu freien Phosphorsäuren hydrolysieren.

Zu den freien Phosphorsäuren kann man auch unmittelbar durch Umsetzung mit einer Mischung aus Phosphorpentoxid und 85 %iger Phosphorsäure (kondensierte Phosphorsäuren) in der Schmelze gelangen. Die Reaktionsmischung kann durch Hydrolyse und Umkristallisieren aus abs. Äthanol aufgearbeitet werden.

II) Herstellung von Verbindungen der allgemeinen Formel mit m = 1 ; A = Sauerstoffatom ; X = Y = Chloratom, Hydroxylrest ; Z = Schwefelatom

Zu derartigen Verbindungen gelangt man, wenn man beispielsweise ein Polyalkylenglycol mit Thiophosphorylchlorid in einem Lösungsmittel umsetzt, z. B. in einem Phosphorsäuretrialkylester, wie Triäthylphosphat. Das erhaltene Halogenid kann man beispielsweise bei einem pH von etwa 7 in Gegenwart von Lithiumhydroxid verseifen. Aus dem Lithiumsalz läßt sich in üblicher Weise die freie Säure freisetzen.

III) Herstellung von Verbindungen der allgemeinen Formel mit m = 1 ; A = Sauerstoffatom ;

X = —O—$C_{1-5}$—alkyl, —O—$C_{1-5}$—alkylen— 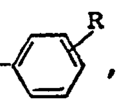 , —O— 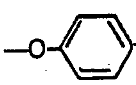 —$NO_2$ —O— 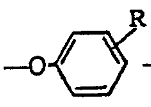 —NH—$C_{1-5}$—alkyl,

—N($C_{1-5}$—alkyl)$_2$, —NH—$C_{1-5}$—alkylen— 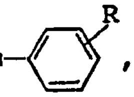 , —N($C_{1-5}$—alkylen— 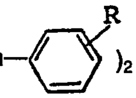 )$_2$ —NH— 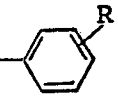

—N( 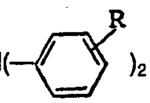 )$_2$

Y = X

R = OH, $C_{1-5}$—alkyl, —O—$C_{1-5}$—alkyl oder fehlt,

Z = O—Atom ;

Die Ester lassen sich dadurch erhalten, daß man entweder die gemäß I) erhaltenen freien Phosphorsäuren in üblicher Weise verestert oder direkt die gemäß I) erhaltenen Phosphorsäurehalogenide mit Alkoholen umsetzt. Wenn man die gemäß I) erhaltenen Phosphorsäurehalogenide mit Aminen umsetzt, gelangt man zu den Phosphorsäureamiden.

IV) Herstellung von Verbindungen der allgemeinen Formel mit m = 1 ; A = Sauerstoffatom ; X = Hydroxylrest ; Y = Alkylthiorest mit 1 bis 5 Kohlenstoffatomen, Aminoalkylthiorest mit 2 bis 5 Kohlenstoffatomen, Carboxyalkylthiorest mit 1 bis 5 Kohlenstoffatomen in der Alkylengruppe ; Z = Sauerstoffatom.

Die Verbindungen mit Alkylthioresten kann man beispielsweise dadurch erhalten, daß man die gemäß II) mit endständigen $(LiO)_2P(S)$—O—Resten erhaltenen Verbindungen mit einem Trialkylphosphat

in wässerigem Medium umsetzt. Durch Umsetzung der genannten gemäß II) erhaltenen Verbindungen mit einem Aminoalkylhalogenid mit 2 bis 5 Kohlenstoffatomen bzw. seinem Hydrohalogenid gelangt man zu den Verbindungen mit Aminoalkylthioresten. Beispielsweise kann man mit omega-Bromalkylamin bzw. seinem Hydrobromid arbeiten. Entsprechend gelangt man durch Umsetzung mit einer Halogencarbonsäure mit 1 bis 5 Kohlenstoffatomen in der Alkylenkette, beispielsweise Bromessigsäure, zu den Verbindungen mit Carboxyalkylthioresten. In allen drei Fällen wird also der $(LiO)_2P(S)$—O—Rest am Schwefelatom alkyliert.

V) Herstellung von Verbindungen der allgemeinen Formel mit m = 1 ; A = Schwefelatom ; X = Y = Hydroxylrest ; Z = Sauerstoffatom

Zur Herstellung derartiger Verbindungen kann man ein Polyalkylenglykol mit einem Thionylhalogenid, beispielsweise Thionylbromid, in einem Lösungsmittel in Gegenwart eines Amins umsetzen, z. B. in Toluol in Gegenwart von Triäthylamin. Das erhaltene Dihalogenid mit endständigen Halogenatomen kann man in einer Åkerfeldt-Reaktion zu Verbindungen mit $(O^-)_2P(O)$—S—Resten umsetzen, beispielsweise mit $(LiO)_3PS$ in einem Wasser/DMF-Gemisch. Aus der erhaltenen Verbindung kann man in bekannter Weise die freie Säure gewinnen.

VI) Herstellung von Verbindungen der allgemeinen Formel mit

m = 0 ; X = Y = OH, —O—$C_{1\text{-}5}$—alkyl, —O—$C_{1\text{-}5}$—alkylen— 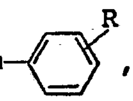 , —O— 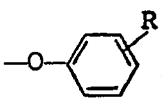 ,

—O—Si($C_{1\text{-}5}$—alkyl$)_3$,
R = OH, $C_{1\text{-}5}$—Alkyl, —O—$C_{1\text{-}5}$—alkyl oder fehlt,
Z = O—Atom.

Zur Herstellung derartiger Verbindungen kann man einen gemäß V) erhaltenen Polyäther mit endständigen Halogenatomen einer Michaelis-Arbusov-Reaktion unterwerfen und mit einem Trialkylphosphit zu Phosphonsäureestern umsetzen. Derartige Ester sind auch durch eine Michaelis-Becker-Reaktion zu erhalten, bei der man einen Polyäther mit endständigen Halogenatomen mit Alkali-, z. B. Natriumphosphorigsäuredialkylestern in einem Lösungsmittel umsetzt, wie Benzol, Toluol oder einem Alkohol. Die erhaltenen Ester kann man direkt mit Säuren, z. B. konzentrierter Salzsäure, zu den freien Phosphonsäuren verseifen. Man kann die freien Phosphonsäuren jedoch auch dadurch erhalten, daß man die Phosphonsäureester zuerst mit einem Trialkylsilylhalogenid umsetzt, z. B. mit Trimethylsilylchlorid oder -bromid, und danach mit Wasser verseift. Diese Verseifung ist gegenüber der Verseifung durch Säuren schonender. Bei der Michaelis-Arbusov-Reaktion kann man anstelle von Trialkylphosphit auch ein gegebenenfalls kernsubstituiertes triphenylphosphit verwenden.

VII) Herstellung von Verbindungen der allgemeinen Formel, bei denen eine der beiden YXP(Z)—O—Gruppen durch eine Alkoxygruppe mit 1 bis 5 Kohlenstoffatomen ersetzt ist.

Zu derartigen Verbindungen gelangt man, wenn man von Polyalkylenglycolen ausgeht, bei denen einer der endständigen OH—Reste durch eine $C_{1\text{-}5}$—Alkylgruppe veräthert ist.

VIII) Herstellung von Salzen von Carbonsäuren und Säuren des Phosphors von Verbindungen der allgemeinen Formel

Sofern derartige Salze nicht bereits vorstehend angesprochen wurden, gelangt man zu ihnen durch übliche Neutralisierung.

Die phosphorhaltigen Polyalkylenglycolderivate, die $HO{-}\overset{\|}{P}{-}$ oder HOOC—Reste aufweisen, lassen sich als flüssige Kationenaustauscher verwenden, beispielsweise zur Reinigung von Proteinen oder Glykoproteinen in Zweiphasensystemen. Im übrigen lassen sich die phosphorhaltigen Polyalkylenglykolderivate in der Kosmetik, Pharmazie und Nahrungsmittelindustrie verwenden, insbesondere die Ester als Pflanzenschutz- und Holzschutzmittel.

Nachstehend wird die Erfindung durch Beispiele näher erläutert.

Beispiel 1

Herstellung von Bisphosphorsäuredichloridpolyäthylenglykolmonoester und Bisphosphorsäurepolyäthylenglykolmonoester

m = 1 ; A = O—Atom ; X = Y = Cl—Atom bzw. OH—Rest ; Z = O—Atom.

$HO{-}CH_2{-}CH_2{-}O{-}(CH_2{-}CH_2{-}O)_p{-}CH_2{-}CH_2{-}OH$ (1) ⟶

$(Cl)_2P(O)—O—CH_2—CH_2—O—(CH_2—CH_2—O)_p—CH_2—CH_2—O—(O)P(Cl)_2$ (2) →
$(HO)_2P(O)—O—CH_2—CH_2—O—(CH_2—CH_2—O)_p—CH_2—CH_2—O—(O)P(OH)_2$ (3)

Es wurden 100 g (16,7 mMol) PEG (mittleres Molekulargewicht 6 000 ; Serva) in einem Rundkolben bei 60 °C im Ölpumpenvakuum 2 h lang getrocknet. Anschließend löste man in 250 ml Phosphorsäuretriäthylester bei 50 °C, der frisch destilliert und über einem Molekularsieb aufbewahrt worden war. Unter Ausschluß von Luftfeuchtigkeit tropfte man innerhalb von 30 min 25,6 g (15,3 ml = 0,166 9 Mol) Phosphoroxychlorid zu. Man rührte noch 6 h lang bei 50 °C, wobei die Reaktionsmischung schwach braun wurde.

Unter Wasserstrahlvakuum und dann Ölpumpenvakuum entfernte man zunächst den Überschuß an Phosphoroxychlorid und den Phoshorsäuretriäthylester aus dem Reaktionsgemisch, der für weitere Reaktionen verwendet wurde. Das Zwischenprodukt (2) wurde durch Zugabe von 500 ml Wasser in 2 h bei Raumtemperatur hydrolysiert. Nach dem Einengen im Rotationsverdampfer erhielt man ein braunes, viskoses Öl, das man in 1 l Wasser löste und mit 20 g Aktivkohle (Merck) behandelte. Nach dem Filtrieren engte man erneut zur Trockne ein, löste den Rückstand in 1 l absolutem, vergälltem Äthanol und behandelte erneut mit 20 g Aktivkohle. Man filtrierte durch eine mit Kieselgur (Serva) beschichtete Fritte und ließ über Nacht bei 4 °C auskristallisieren. Der farblose Kristallbrei wurde abgesaugt, mit Äther (DAB 7) gewaschen und im Vakuumexsikkator getrocknet. Auswaage an trockener Verbindung (3) : 90 g.

Charakterisierung :

1. Aufnahme der Titrationskurve ; OH—Reste zu 90 bis 100 % durch Phosphatreste substituiert.
2. $^{31}P$-Spektrum ; 1 Signal bei 0,1 ppm.
3. Phosphatbestimmung nach Aufschluß.
4. Die wässerige Lösung der Verbindung (3) enthielt kein Chlorid und kein freies Phosphat.

Beispiel 2

Herstellung von Bisphosphorsäuredichloridpolyäthylenglykolmonoester und Bisphosphorsäurepolyäthylenglykolmonoester

m = 1 ; A = O—Atom ; X = Y = Cl—Atom bzw. OH—Rest ; Z = O—Atom.

Es wurden 130 g (84,4 mMol) PEG (mittleres Molekulargewicht 1 540 ; Riedel de Haen) 2 h lang bei 50 °C unter Ölpumpenvakuum getrocknet und danach in 250 ml Triäthylphosphat bei 40 °C gelöst. Unter Feuchtigkeitsausschluß und unter Rühren tropfte man 40 ml (425 mMol) Phosphoroxychlorid innerhalb von 30 min zu. Nach 3 h zog man unter Wasserstrahlvakuum bei 40 °C das überschüssige Phosphoroxychlorid 1 h lang und anschließend unter Ölpumpenvakuum das Triäthylphosphat 3 h lang ab. Man hydrolysierte mit 2 l Wasser bei 4 °C 5 h lang. Man engte im Rotationsverdampfer ein, versetzte den öligen Rückstand dreimal mit je 100 ml Wasser und engte erneut ein. Den schwach gelblichen Rückstand löste man in 1 l absolutem, vergälltem Äthanol und ließ über Nacht bei 4 °C stehen. Nach dem Absaugen des Kristallbreies wusch man mit kaltem Äthanol und Äther und trocknete im Vakuumexsikkator. Auswaage an trockenem Phosphat-Polyäthylenglykol-Phosphat : 122 g.

Charakterisierung : vergleiche Beispiel 1

1. $^{31}P$-Spektrum : 0,2 ppm
2. Die Substanz enthielt kein freies Phosphat und war chloridfrei.

Beispiel 3

Herstellung von Verbindungen der allgemeinen Formel mit

m = 1 ; A = O—Atom ; X = Y = Cl—Atom oder OH—Rest ; Z = S—Atom ; bzw. deren Salze

$HO—CH_2—CH_2—O—(CH_2—CH_2—O)_p—CH_2—CH_2—OH$ (1) →
$(Cl)_2P(S)—O—CH_2—CH_2—O—(CH_2—CH_2—O)_p—CH_2—CH_2—O—(S)P(Cl)_2$ (2) →
$(O^-)_2P(S)—O—CH_2—CH_2—O—(CH_2—CH_2—O)_p—CH_2—CH_2—O—(S)P(O^-)_2$ (3)
2 oder 4 $Li^+$

Es wurden 60 g (10 mMol) PEG (mittleres Molekulargewicht 6 000 ; Serva) bei 50 °C 2 h lang unter Ölpumpenvakuum getrocknet. Nach dem Lösen in 150 ml Triäthylphosphat wurden unter Feuchtigkeitsausschluß und Rühren bei 50 °C 5,2 ml (50 mMol) Thiophosphorylchlorid innerhalb von 30 min zugetropft. Nachdem man 5 h lang gerührt hatte, wurde überschüssiges Thiophosphorylchlorid unter Wasserstrahlvakuum (1 h) und anschließend Triäthylphosphat unter Ölpumpenvakuum abgezogen. Das Zwischenprodukt (2) wurde mit 300 ml Wasser beim pH 7 durch Zugeben einer 2 m LiOH—Lösung hydrolysiert (pH—Stat). Nach dem Einengen in einem Rotationsverdampfer wurde der Rückstand in 600 ml vergälltem absoluten Äthanol gelöst und über Nacht bei 4 °C stehen gelassen. Der Kristallbrei wurde

abgesaugt und mit eiskaltem Äthanol und Äther gewaschen. Nach dem Trocknen im Vakuumexsikkator erhielt man 55 g Polyäthylenglykolthionphosphat (3) als Lithiumsalz.

Charakterisierung :
1. Die Verbindung (3) enthielt kein freies Thionophosphat und war chloridfrei.
2. Substitutionsgrad 90 bis 100 %.
3. $^{31}P$-Spektrum : 43,41 ppm

Beispiel 4

Herstellung von Verbindungen der allgemeinen Formel mit

m = 1 ; A = O—Atom ; X = OH—Rest ; Y = Methylthiorest, Carboxymethylthiorest bzw. omega-Aminoäthylthiorest ; Z = O—Atom ; bzw. deren Salze

$(O^-)_2P(S)—O—CH_2—CH_2—O—(CH_2—CH_2—O)_p—CH_2—CH_2—O—(S)_p(O^-)_2$ (1) ⟶
2 oder 4 $Li^+$
$CH_3S—P(O^-)$ (O)—O—$CH_2—CH_2—O—(CH_2—CH_2—O)_p—CH_2—CH_2—O$—(O) $(O^-)P—SCH_3$ (2)
2 $Li^+$

A. Es wurden 6 g (ca. 1 mMol) der Verbindung (1) in 50 ml Wasser gelöst und mit 10 ml Trimethylphosphat versetzt. Während der Reaktion wurde der pH-Wert bei 7,5 gehalten. Der Ablauf der Reaktion ließ sich gut mit einer Silbernitratlösung verfolgen. Während die Verbindung (1) mit Silbernitrat in salpetersaurer Lösung unter Braunfärbung reagiert, ergibt die Verbindung (2) keine Färbung. Die Reaktion war bei Raumtemperatur nach ca. 4 h beendet. Man engte im Rotationsverdampfer unter Wasserstrahl- und Ölpumpenvakuum ein und kristallisierte den Rückstand aus vergälltem absoluten Äthanol. Nach Absaugen des Kristallbreis, Waschen mit Äther und Trocknen in einem Vakuumexsikkator erhielt man 5 g der Verbindung (2). Die Umsetzung war quantitativ.

Charakterisierung :
$^{31}P$-Spektrum : Die Struktur wurde eindeutig durch ein Signal bei 22,32 ppm belegt.

$(O^-)_2P(S)—O—CH_2—CH_2—O—(CH_2—CH_2—O)_p—CH_2—CH_2—O—(S)P(O^-)_2$ (1) ⟶
2 oder 4 $Li^+$
$HOOC—CH_2—S—P(O^-)$ (O)—O—$CH_2—CH_2—O—(CH_2—CH_2—O)_p—CH_2—CH_2—O$—(O) $(O^-)P—S—CH_2—COOH$ (3)
2 $Li^+$

B. Es wurden 3 g (ca. 0,5 mMol) der Verbindung (1) in 100 ml Wasser gelöst und mit 500 mg Bromessigsäure bei Raumtemperatur versetzt. Der pH-Wert der Reaktionslösung wurde bei 7,5 gehalten. Die Reaktion war nach ca. 2 h beendet (keine Braunfärbung mit Silbernitratlösung). Der Ansatz wurde durch Dialyse entsalzt. Nach dem Lyophilisieren der wässerigen Lösung erhielt man etwa 2,5 g der Verbindung (3). Die Umsetzung war quantitativ.

Charakterisierung :
$^{31}P$-Spektrum : Die Struktur wurde eindeutig durch ein Signal bei 20,5 ppm belegt.

$(O^-)_2P(S)—O—CH_2—CH_2—O—(CH_2—CH_2—O)_p—CH_2—CH_2—O—(S)P(O^-)_2$ (1) ⟶
2 oder 4 $Li^+$
$H_2N—CH_2—CH_2—S—P(O^-)$ (O)—O—$CH_2—CH_2—O—(CH_2—CH_2—O)_p—CH_2—CH_2—O$—(O) $(O^-)P—S—CH_2—CH_2—NH_2$ (4)
2 $Li^+$

C. Es wurden 6 g (ca. 1 mMol) der Verbindung (1) in 100 ml Wasser gelöst und mit 470 mg 2-Bromäthylammoniumbromid bei Raumtemperatur versetzt. Der pH-Wert der Reaktionslösung wurde durch Zugeben von 0,2 n NaOH bei 7,5 gehalten. Die Reaktion war nach 5 h beendet (keine Braunfärbung mit Silbernitratlösung). Nach dem Einengen im Rotationsverdampfer wurde der Rückstand aus vergälltem absoluten Äthanol kristallisiert. Nach Absaugen des Kristallbreis, Waschen mit Äther und Trocknen im Vakuumexsikkator erhielt man ca. 5 g der Verbindung (4). Die Reaktion war wiederum quantitativ.

Charakterisierung :
$^{31}P$-Spektrum : Die Struktur wurde eindeutig durch ein Signal bei 19,2 ppm belegt.

Beispiele 5 bis 8

Herstellung von Verbindungen der allgemeinen Formel mit

m = 0 ; X = Y = Äthoxyrest ; Z = O—Atom

$HO-CH_2-CH_2-O-(CH_2-CH_2-O)_p-CH_2-CH_2-OH \rightarrow$
$Br-CH_2-CH_2-O-(CH_2-CH_2-O)_p-CH_2-CH_2-Br$ (1) $\rightarrow$
$(C_2H_5O)_2P(O)-CH_2-CH_2-O-(CH_2-CH_2-O)_p-CH_2-CH_2-(O)P(OH_5C_2)_2$ (2)

Es wurden Polyäthylenglykole (Molekulargewicht 1 540, 6 000, 10 000 bzw. 20 000) in Toluol in Gegenwart von Triäthylamin mit Thiolylbromid in bekannter Weise umgesetzt ; vgl. Johansson, G., Biochim. Biophys. Acta, 222 bis 381 (1970) und Johansson, G., Hartman, A. und Albertsson, P.-A., Eur. J. Biochem., 33, 379 bis 386 (1973).

Es wurden 6 g Bisbrompolyäthylenglykol (mittleres Molekulargewicht 6 000 ; Serva ; bzw. eine entsprechende Menge der anderen Bisbromverbindungen der vorstehend angeführten Polyäthylenglykole) mit 10 ml Triäthylphosphit (Überschuß) in einem Rundkolben versetzt. Man erhitzte 24 h lang unter Rückfluß (Luftkühlung) und Feuchtigkeitsausschluß ; danach ließ man Abkühlen und versetzte mit 50 ml Äther. Man filtrierte auf einer Fritte ab und entfernte überschüssiges Triäthylphosphit durch Waschen mit Äther. Nach dem Trocknen in einem Vakuumexsikkator erhielt man ca. 6 g der Verbindung (2). Spuren von Triäthylphosphit wurden unter Ölpumpenvakuum entfernt. Die Umsetzung war quantitativ. Der Bromgehalt betrug nach der Umsetzung weniger als 0,05 %.

Charakterisierung der Verbindungen (3) :

1. $^{31}P$-Spektrum :

| Mittleres-PEG-Molekulargewicht | 1 540 | 6 000 | 10 000 | 20 000 |
|---|---|---|---|---|
| Signal (ppm) | 32,1 | 32,14 | 32,1 | 32,1 |

2. Substitutionsgrad : abhängig vom Bromierungsgrad, der bei den einzelnen Polyäthylenglykolen zwischen 60 und 100 % lag.

## Beispiel 9

Herstellung von Verbindungen der allgemeinen Formel mit

m = 0 ; X = Y = Äthoxyrest ; Z = O—Atom ; eine der beiden YXP (Z)—$A_m$—Gruppen ist durch eine Methoxygruppe ersetzt.

$HO-CH_2-CH_2-O-(CH_2-CH_2-O)_p-CH_2-CH_2-O-CH_3 \rightarrow$
$Br-CH_2-CH_2-O-(CH_2-CH_2-O)_p-CH_2-CH_2-O-CH_3$ (1) $\rightarrow$
$(C_2H_5O)_2P(O)-CH_2-CH_2-O-(CH_2-CH_2-O)_p-CH_2-CH_2-O-CH_3$ (2)

Es wurde gemäß den Beispielen 5 bis 8 mit der Ausnahme gearbeitet, daß anstelle von Polyäthylenglykol von Monomethoxypolyäthylenglykol ausgegangen wurde (mittleres Molekulargewicht 5 000).

Charakterisierung der Verbindung :
$^{31}P$-Spektrum : 32,1 ppm

## Beispiele 10 bis 13

Herstellung von Verbindungen der allgemeinen Formel mit

m = 0 ; X = Y = Trimethylsilyloxy- oder Hydroxyl-Rest,
Z = O—Atom

$(C_2H_5O)_2P(O)-CH_2-CH_2-O-(CH_2-CH_2-O)_p-CH_2-CH_2-(O)P(OH_5C_2)_2$ (2) $\rightarrow$
$[(CH_3)_3SiO]_2P(O)-CH_2-CH_2-O-(CH_2-CH_2-O)_p-CH_2-CH_2-(O)P[OSi(CH_2)_3]_2$ (3) $\rightarrow$
$(HO)_2P(O)-CH_2-CH_2-C-(CH_2-CH_2-O)_p-CH_2-CH_2-(O)P(OH)_2$ (4)

Es wurden 24 g der Verbindung (2) der Beispiele 5 bis 8 (mittleres PEG-Molekulargewicht 6 000 ; entsprechende Mengen der analogen anderen Verbindungen (2) der Beispiele 5 bis 8) in 150 ml wasserfreiem Dichlormethan gelöst und mit 3,5 ml Bromtrimethylsilan (Überschuß ; Aldrich) unter Feuchtigkeitsausschluß versetzt. Man ließ 12 h, 2 d oder länger bei Raumtemperatur stehen, engte dann im Rotationsverdampfer ein und versetzte mit 500 ml Wasser. Man rührte 5 h lang oder über Nacht, engte erneut ein und kristallisierte aus Äthanol um. Die Ausbeute an Verbindung (4) betrug 17 g (mittleres PEG-Molekulargewicht 6 000).

Charakterisierung :

1. $^{31}P$-Spektren :

| Mittleres PEG-Molekulargewicht | 1 540 | 6 000 | 10 000 | 20 000 |
|---|---|---|---|---|
| Signal (ppm) | 26,5 | 25,9 | 25,9 | 25,9 |

2. $^{13}C$-Spektrum : Für die Verbindungen (4) mit einem mittleren PEG-Molekulargewicht von 1 540 wurde die Struktur durch ein derartiges Spektrum zusätzlich bestätigt.

## Beispiel 14

Herstellung von Verbindungen der allgemeinen Formel mit

m = 0 ; X = Y = Trimethylsiloxy- oder Hydroxylgruppe ; Z = O—Atom ; eine der beiden YXP(Z)—$A_m$—Gruppen ist durch eine Methoxygruppe ersetzt.

Es wurden die Beispiele 10 bis 13 mit der Ausnahme wierderholt, daß Verbindung (3) von Beispiel 9 eingesetzt wurde.

Charakterisierung :
$^{31}P$-Spektrum : 26,00 ppm

## Beispiel 15

Herstellung von Verbindungen der allgemeinen Formel mit

m = 1 ; A = S—Atom ; X = Y = OH ; Z = O—Atom ; bzw. deren Salze

$Br—CH_2—CH_2—O—(CH_2—CH_2—O)_p—CH_2—CH_2—Br$ (1) ⟶
$(O^-)_2P(O)—S—CH_2—CH_2—O—(CH_2—CH_2—O)_p—CH_2—CH_2—S—(O)P(O^-)_2$ (3)

Es wurden 1,6 g (ca. 1 mMol) der Verbindung (1) in 20 ml Wasser gelöst, mit 5 ml Dimethylformamid und 250 mg Trilithiumthionphosphat versetzt (pH 11,7). Der Ablauf der Reaktion wurde mit Silbernitrat in Salpetersäurelösung verfolgt. Nach 24 h wurden erneut 150 mg Trilithiumthionphosphat zugegeben (pH 10,6). Nach 3 Tagen (pH 8,5) wurde im Rotationsverdampfer zur Trockne eingeengt, die Verbindung (3) in 50 ml Dichlormethan gelöst und vom Salz abgetrennt. Die Dichlormethanlösung wurde eingeengt und der Rückstand aus Wasser lyophilisiert. Die Ausbeute betrug 1,2 g.

Charakterisierung :
$^{31}P$-Spektrum : Die Struktur der Verbindung (3) wurde durch ein Signal bei 16,4 ppm eindeutig belegt.

**Patentansprüche** (für die Vertragsstaaten : CH, DE, FR, GB, LI, LU, NL, SE)

1. Verbindungen der allgemeinen Formel

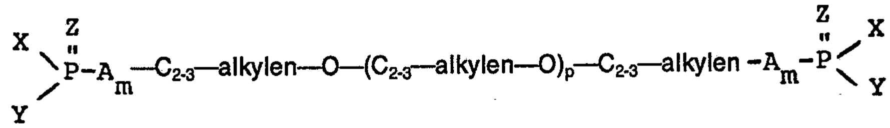

p = 1 bis 800 und
(a1) m = 1,
A = O—Atom,
X = Cl oder OH und Y = X oder —O—$C_{1-5}$—alkyl,
Z = O— oder S—Atom ; wobei für X = OH, Y = —O—$C_{1-5}$—alkyl und Z = O—Atom p kleiner oder gleich 100 ausgenommen ist, oder
(a2) m = 1,
A = O—Atom,

X = —O—$C_{1-5}$—alkyl, —O—$C_{1-5}$—alkylen— 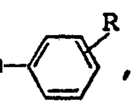 , —O— 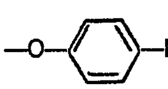 —$NO_2$ —O— 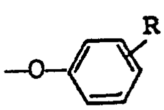

—NH—$C_{1-5}$—alkyl, —N($C_{1-5}$—alkyl)$_2$, —NH—$C_{1-5}$—alkylen— 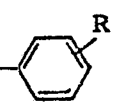 , —N($C_{1-5}$—alkylen— 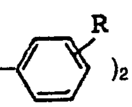 )$_2$ ,

—NH— 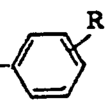 —N(— 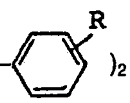 )$_2$

Y = X oder —O—$C_{1-5}$—alkyl,
R = OH, $C_{1-5}$—alkyl, —O—$C_{1-5}$—alkyl oder fehlt,
Z = O—Atom ; wobei für X = —N($C_{1-5}$—alkyl)$_2$ oder —O—$C_{1-5}$—alkyl p kleiner oder gleich 7 ausgenommen ist, oder

(a3) m = 1,
A = O—Atom,
X = OH,
Y = —S—$C_{1-5}$—alkyl, —S—amino—$C_{2-5}$—alkyl, S—carboxy—$C_{1-5}$—alkyl,
Z = O—Atom ; oder

(b) m = 1,
A = S—Atom,
X = Y = OH,
Z = O—Atom ; oder

(c) m = 0,
X = Y = OH, —O—$C_{1-5}$—alkyl, —O—$C_{1-5}$—alkylen—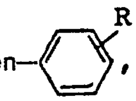, —O—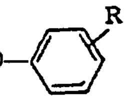—O—Si($C_{1-5}$—alkyl)$_3$,
R = OH, $C_{1-5}$—Alkyl, —O—$C_{1-5}$—alkyl oder fehlt,
Z = O—Atom,

und deren Derivate, bei denen einer der beiden 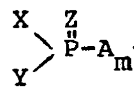-Gruppen durch eine $C_{1-5}$—Alkoxygruppe ersetzt ist
und die Alkalimetallderivate und Salze mit Ammoniak und Aminen der Säuren des Phosphors und der Carbonsäuren.

2. Verbindungen nach Anspruch 1, dadurch gekennzeichnet, daß in der allgemeinen Formel gemäß Anspruch 1 $C_{2-3}$—Alkylen = —$CH_2$—$CH_2$, —$CH_2$—$CH_2$—$CH_2$—, —$CH_2$—CH($CH_3$)— oder deren Gemische ; p = 3 bis 250 und vorzugsweise 30 bis 160.

3. Verbindungen nach Anspruch 1 oder 2, dadurch gekennzeichnet, daß in der allgemeinen Formel gemäß Anspruch 1 —O—$C_{1-5}$—alkyl = Methoxy, Äthoxy oder Propoxy, —O—$C_{1-5}$—alkylen—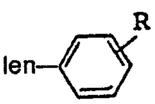 = —O—$C_1$—alkylen—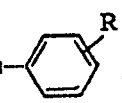, —NH—$C_{1-5}$—alkyl = Methylamino, Äthylamino oder Propylamino, —N($C_{1-5}$—alkyl)$_2$ = Dimethylamino, Diäthylamino oder Dipropylamino, —NH—$C_{1-5}$—alkylen—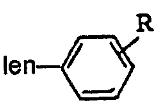 = —NH—$C_1$—alkylen—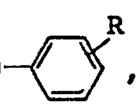, —N($C_{1-5}$—alkylen—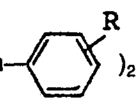)$_2$ = —N($C_1$—alkylen—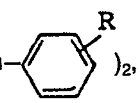)$_2$, —S—$C_{1-5}$—alkyl = —S—$C_{1-3}$—alkyl, —S—amino—$C_{2-5}$—alkyl = —S—amino—$C_{2-3}$—alkyl, —S—carboxy—$C_{1-5}$—alkyl = —S—carboxy—$C_{1-3}$—alkyl und/oder —O—Si($C_{1-5}$—alkyl)$_3$ = —O—Si($CH_3$)$_3$ für X und/oder Y.

4. Verbindungen nach einem der vorhergehenden Ansprüche, dadurch gekennzeichnet, daß in der allgemeinen Formel gemäß Anspruch 1 $C_{1-5}$—Alkyl = Methyl, Äthyl oder Propyl und/oder —O—$C_{1-5}$—alkyl = Methoxy, Äthoxy oder Propoxy für R.

5. Verbindungen nach einem der vorhergehenden Ansprüche, gekennzeichnet durch die Ammonium—, Li—, Na— oder K—Salze, vorzugsweise der Säuren des Phosphors.

**Patentansprüche** (für den Vertragsstaat AT)

1. Verfahren zur Herstellung von Polyalkylenglykol-biphosphorsäuremonoestern, Polyalkylenglykolbisphosphorsäuren und deren Derivaten, dadurch gekennzeichnet, daß man zur Herstellung von Verbindungen der allgemeinen Formel

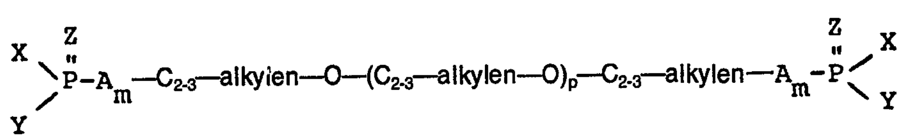

p = 1 bis 800 und
(a1) m = 1,

A = O—Atom,
X = Cl oder OH und Y = X oder —O—$C_{1-5}$—alkyl,
Z = O— oder S—Atom ; wobei für X = OH, Y = —O—$C_{1-5}$—alkyl und
Z = O—Atom p kleiner oder gleich 100 ausgenommen ist, oder

(a2) m = 1,
A = O—Atom,

X = —O—$C_{1-5}$—alkyl, —O—$C_{1-5}$—alkylen— 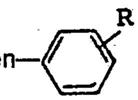, —O— 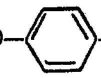 —$NO_2$ —O— 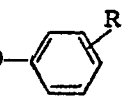

—NH—$C_{1-5}$—alkyl, —N($C_{1-5}$—alkyl)$_2$, —NH—$C_{1-5}$—alkylen— 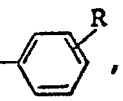, —N($C_{1-5}$—alkylen— 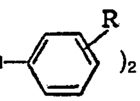 )$_2$

—NH— 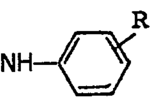 —N( 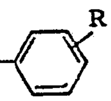 )$_2$

Y = X oder —O—$C_{1-5}$—alkyl,
R = OH, $C_{1-5}$—alkyl, —O—$C_{1-5}$—alkyl oder fehlt,
Z = O—Atom ; wobei für X = —N($C_{1-5}$—alkyl)$_2$ oder —O—$C_{1-5}$—alkyl p kleiner oder gleich 7 ausgenommen ist, oder

(a3) m = 1,
A = O—Atom,
X = OH,
Y = —S—$C_{1-5}$—alkyl, —S—amino—$C_{2-5}$—alkyl, S—carboxy—$C_{1-5}$—alkyl,
Z = O—Atom ; oder

(b) m = 1,
A = S—Atom,
X = Y = OH,
Z = O—Atom ; oder

(c) m = 0,

X = Y = OH, —O—$C_{1-5}$—alkyl, —O—$C_{1-5}$—alkylen— 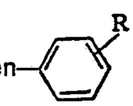, —O— 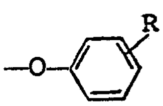 —O—Si($C_{1-5}$—alkyl)$_3$,

R = OH, $C_{1-5}$—Alkyl, —O—$C_{1-5}$—alkyl oder fehlt,
Z = O—Atom,

und deren Derivaten, bei denen einer der beiden 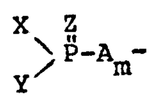 Gruppen durch eine $C_{1-5}$—Alkoxygruppe ersetzt ist,
und deren Alkalimetallderivaten und Salzen mit Ammoniak und Aminen der Säuren des Phosphors und der Carbonsäure,

(a1) mit
m = 1,
A = O—Atom,
X = Cl oder OH und Y = X oder —O—$C_{1-5}$—alkyl,
Z = O— oder S—Atom ; wobei für X = OH, Y = —O—$C_{1-5}$—alkyl und
Z = O—Atom p kleiner oder gleich 100 ausgenommen ist,

ein Polyalkylenglykol mit Phosphoroxychlorid oder Pyrophosphorylchlorid mit oder ohne Lösungsmittel in Gegenwart eines Säurefängers umsetzt, die erhaltenen Phosphorsäurehalogenide gegebenenfalls mit Wasser oder zuerst mit der halben stöchiometrischen Menge eines $C_{1-5}$—Alkanols und danach mit Wasser zu freier Phosphorsäure hydrolysiert ; oder
ein Polyalkylenglykol mit einem Gemisch aus Phosphorpentoxid und 85-proz. Phosphorsäure (kondensierte Phosphorsäuren) schmilzt ; oder
ein Polyalkylenglykol mit Thiophosphorylchlorid in einem Lösungsmittel umsetzt, bei einem pH-Wert von etwa 7 in Gegenwart von Lithiumhydroxid verseift und in üblicher Weise die freie Säure freisetzt ;

(a2) mit
m = 1,
A = O—Atom,

X = —O—$C_{1\text{-}5}$—alkyl, —O—$C_{1\text{-}5}$—alkylen—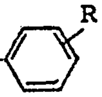, —O—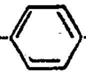—$NO_2$ —O—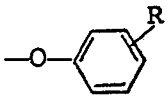 —NH—$C_{1\text{-}5}$

—alkyl, —N($C_{1\text{-}5}$—alkyl)$_2$, —NH—$C_{1\text{-}5}$—alkylen—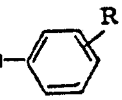, —N($C_{1\text{-}5}$—alkylen—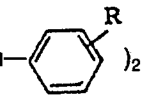)$_2$ —NH—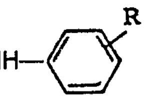
—NH—

—N(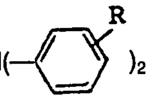)$_2$

Y = X oder —O—$C_{1\text{-}5}$—alkyl,
R = OH, $C_{1\text{-}5}$—alkyl, —O—$C_{1\text{-}5}$—alkyl oder fehlt,
Z = O—Atom ; wobei für X = —N($C_{1\text{-}5}$—alkyl)$_2$ oder —O—$C_{1\text{-}5}$—alkyl p kleiner oder gleich 7 ausgenommen ist,
die gemäß (a1) erhaltenen freien Phosphorsäuren in üblicher Weise verestert oder direkt die gemäß (a1) erhaltenen Phosphorsäurehalogenide mit Alkoholen oder Aminen umsetzt ;

(a3) mit
m = 1,
A = O—Atom,
X = OH,
Y = —S—$C_{1\text{-}5}$—alkyl, —S—amino—$C_{2\text{-}5}$—alkyl, —S—carboxy—$C_{1\text{-}5}$—alkyl,
Z = O—Atom,
ein Polyalkylenglykol mit Thiophosphorylchlorid in einem Lösungsmittel umsetzt, bei einem pH-Wert von etwa 7 in Gegenwart von Lithiumhydroxid verseift, die erhaltenen Verbindungen mit endständigen $(LiO)_2P(S)$—O—Resten mit einem Trialkylphosphat in wäßrigem Medium, einem Aminoalkylhalogenid mit 2 bis 5 Kohlenstoffatomen bzw. seinem Hydrohalogenid oder einer Halogencarbonsäure mit 1 bis 5 Kohlenstoffatomen in der Alkylkette umsetzt ;

(b) mit
m = 1,
A = S—Atom,
X = Y = OH,
Z = O—Atom,
ein Polyalkylenglykol mit einem Thionylhalogenid in einem Lösungsmittel in Gegenwart eines Amins umsetzt, das erhaltene Dihalogenid mit endständigen Halogenatomen in einer Åkerfeldt-Reaktion zu Verbindungen mit $(O^-)_2P(O)$—S—Resten umsetzt und gegebenenfalls in bekannter Weise die freie Säure gewinnt ;

(c) mit
m = 0,

X = Y = OH, —O—$C_{1\text{-}5}$—alkyl, —O—$C_{1\text{-}5}$—alkylen—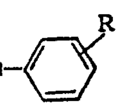, —O—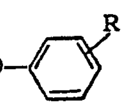 —O—Si($C_{1\text{-}5}$—alkyl)$_3$,

R = OH, $C_{1\text{-}5}$—Alkyl, —O—$C_{1\text{-}5}$—alkyl oder fehlt,
Z = O—Atom,
einen gemäß (b) erhaltenen Polyether mit endständigen Halogenatomen einer Michaelis-Arbusov-Reaktion unterwirft und mit einem gegebenenfalls kernsubstituierten Triphenylphosphit oder einem Trialkylphosphit zu Phosphonsäureestern umsetzt oder in einer Michaelis-Becker-Reaktion einen Polyether mit endständigen Halogenatomen mit Alkaliphosphorigsäuredialkylestern in einem Lösungsmittel umsetzt und gegebenenfalls die erhaltenen Ester direkt mit Säuren zu freien Phosphonsäuren verseift oder die Phosphonsäureester zuerst mit einem Trialkylsilylhalogenid umsetzt und danach mit Wasser verseift,
wobei man zur Herstellung von Verbindungen der allgemeinen Formel, bei denen eine der beiden YXP(Z)—O—Gruppen durch eine $C_{1\text{-}5}$—Alkoxygruppe ersetzt ist, bei dem Verfahren gemäß (a1), (a2), (a3), (b) oder (c) von einem Polyalkylenglykol ausgeht, dessen einer endständiger OH—Rest durch eine $C_{1\text{-}5}$—Alkylgruppe veräthert ist, und
wobei man zur Herstellung von Verbindungen der allgemeinen Formel in Form von Salzen die gemäß (a1), (a2), (a3), (b) oder (c) erhaltenen Säuren der allgemeinen Formel in üblicher Weise neutralisiert.

2. Verfahren nach Anspruch 1, dadurch gekennzeichnet, daß in der allgemeinen Formel gemäß Anspruch 1 $C_{2\text{-}3}$—Alkylen = —$CH_2$—$CH_2$, —$CH_2$—$CH_2$—$CH_2$—, —$CH_2$—$CH(CH_3)$— oder deren Gemische ; p = 3 bis 250 und vorzugsweise 30 bis 160.

3. Verfahren nach Anspruch 1 oder 2, dadurch gekennzeichnet, daß in der allgemeinen Formel gemäß Anspruch 1 —O—$C_{1-5}$—alkyl = Methoxy, Äthoxy oder Propoxy, —O—$C_{1-5}$—alkylen—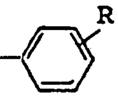 = —O—$C_1$—alkylen—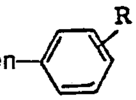, —NH—$C_{1-5}$—alkyl = Methylamino, Äthylamino oder Propylamino, —N($C_{1-5}$—alkyl)$_2$ = Dimethylamino, Diäthylamino oder Dipropylamino, —NH—$C_{1-5}$—alkylen—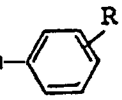 = —NH—$C_1$—alkylen—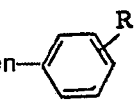, —N($C_{1-5}$—alkylen—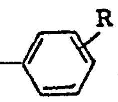)$_2$ = —N($C_1$—alkylen—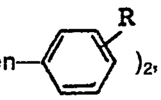)$_2$, —S—$C_{1-5}$—alkyl = —S—$C_{1-3}$—alkyl, —S—amino—$C_{2-5}$—alkyl = —S—amino—$C_{2-3}$—alkyl, —S—carboxy—$C_{1-5}$—alkyl = —S—carboxy—$C_{1-3}$—alkyl und/oder —O—Si($C_{1-5}$—alkyl)$_3$ = —O—Si($CH_3$)$_3$ für X und/oder Y.

4. Verbindungen nach einem der vorhergehenden Ansprüche, dadurch gekennzeichnet, daß in der allgemeinen Formel gemäß Anspruch 1 $C_{1-5}$—Alkyl = Methyl, Äthyl oder Propyl und/oder —O—$C_{1-5}$—alkyl = Methoxy, Äthoxy oder Propoxy für R.

5. Verfahren nach einem der vorhergehenden Ansprüche, dadurch gekennzeichnet, daß man Säuren der allgemeinen Formel gemäß Anspruch 1 zu den Ammonium—, Li—, Na— oder K—Salzen neutralisiert.

**Claims** (for the Contracting States : CH, DE, FR, GB, LI, LU, NL, SE)

1. Compounds of the general formula

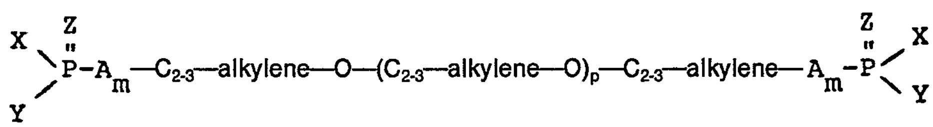

p = from 1 to 800, and

(a1) m = 1,

A = O atom,

X = Cl or OH and Y = X or —O—$C_{1-5}$—alkyl,

Z = O or S atom ; but where X = OH, Y = —O—$C_{1-5}$—alkyl and Z = O atom, p cannot be less than or equal to 100, or

(a2) m = 1,

A = O atom,

X = —O—$C_{1-5}$—alkyl, —O—$C_{1-5}$—alkylene—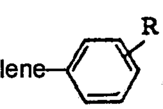, —O—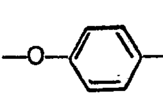—$NO_2$, —O—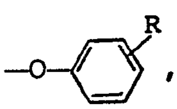, —NH—$C_{1-5}$—alkyl, —N($C_{1-5}$—alkyl)$_2$, —NH—$C_{1-5}$—alkylene—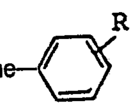, —N($C_{1-5}$—alkylene—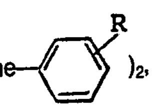)$_2$, —NH—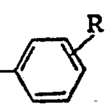, —N(—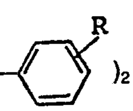)$_2$

Y = X or —O—$C_{1-5}$—alkyl,

R = OH, $C_{1-5}$—alkyl, —O—$C_{1-5}$—alkyl or is absent,

Z = O atom ; but where X = —N($C_{1-5}$—alkyl)$_2$ or —O—$C_{1-5}$—alkyl, p cannot be less than or equal to 7, or

(a3) m = 1,

A = O atom,

X = OH,

Y = —S—$C_{1-5}$—alkyl, —S—amino—$C_{2-5}$—alkyl, S—carboxy—$C_{1-5}$—alkyl,
Z = O atom ; or
(b) m = 1,
A = S atom,
X = Y = OH,
Z = O atom ; or
(c) m = 0,

X = Y = OH, —O—$C_{1-5}$—alkyl, —O—$C_{1-5}$—alkylene— 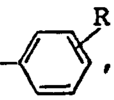 , —O— 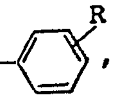 , —O—Si($C_{1-5}$—alkyl)$_3$,

R = OH, $C_{1-5}$—alkyl, —O—$C_{1-5}$—alkyl or is absent,
Z = O atom,

and derivatives thereof, in which one of the two 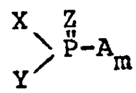 groups has been replaced by a $C_{1-5}$—alkoxy group,
and the alkali metal derivatives and salts with ammonia and amines of the acids of phosphorus and of carboxylic acids.

2. Compounds according to claim 1, characterised in that in the general formula according to claim 1, $C_{2-3}$—alkylene = —$CH_2$—$CH_2$—, —$CH_2$—$CH_2$—$CH_2$—, —$CH_2$—$CH(CH_3)$— or mixtures thereof ; p = from 3 to 250 and preferably from 30 to 160.

3. Compounds according to claim 1 or 2, characterised in that in the general formula according to claim 1, —O—$C_{1-5}$—alkyl = methoxy, ethoxy or propoxy,

—O—$C_{1-5}$—alkylene— 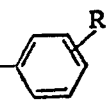 = —O—$C_1$—alkylene— 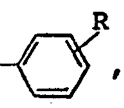 , —NH—$C_{1-5}$—alkyl = methylamino, ethylamino or propylamino, —N($C_{1-5}$—alkyl)$_2$ = dimethylamino, diethylamino or dipropylamino,

—NH—$C_{1-5}$—alkylene— 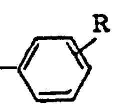 = —NH—$C_1$—alkylene— 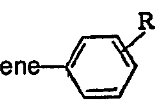 , —N($C_{1-5}$—alkylene— 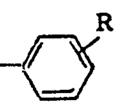 )$_2$ =

—N($C_1$—alkylene— 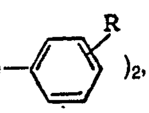 )$_2$, —S—$C_{1-5}$—alkyl = —S—$C_{1-3}$—alkyl, —S—amino—$C_{2-5}$—alkyl = —S—amino—$C_{2-5}$—alkyl, —S—carboxy—$C_{1-5}$—alkyl = —S—carboxy—$C_{1-3}$—alkyl and/or —O—Si($C_{1-5}$—alkyl)$_3$ = —O—Si($CH_3$)$_3$ for X and/or Y.

4. Compounds according to any one of the preceding claims, characterised in that in the general formula according to claim 1, $C_{1-5}$—alkyl = methyl, ethyl or propyl and/or —O—$C_{1-5}$—alkyl = methoxy, ethoxy or propoxy for R.

5. Compounds according to any one of the preceding claims, characterised by the ammonium, Li, Na or K salts, preferably of acids of phosphorus.

**Claims** (for the Contracting State AT)

1. Process for manufacturing bisphosphoric acid polyalkylene glycol monoesters, polyalkylene glycol bisphosphoric acids and their derivatives, characterized in that for the manufacture of compounds of the general formula

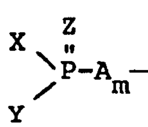 —$C_{2-3}$—alkylene—O—($C_{2-3}$—alkylene—O)$_p$—$C_{2-3}$—alkylene— 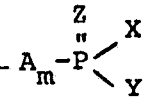

p = from 1 to 800, and
(a1) m = 1,
A = O atom,

X = Cl or OH and Y = X or —O—$C_{1-5}$—alkyl,

Z = O or S atom ; but where X = OH, Y = —O—$C_{1-5}$—alkyl and Z = O atom, p cannot be less than or equal to 100, or

(a2) m = 1,

A = O atom,

X = —O—$C_{1-5}$—alkyl, —O—$C_{1-5}$—alkylene—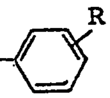, —O—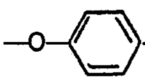—$NO_2$, —O—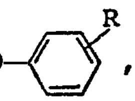,

—NH—$C_{1-5}$—alkyl, —N($C_{1-5}$—alkyl)$_2$, —NH—$C_{1-5}$—alkylene—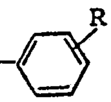, —N($C_{1-5}$—alkylene—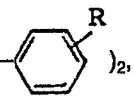)$_2$,

—NH—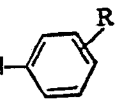, —N(—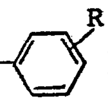)$_2$

Y = X or —O—$C_{1-5}$—alkyl,

R = OH, $C_{1-5}$—alkyl, —O—$C_{1-5}$—alkyl or is absent,

Z = O atom ; but where X = —N($C_{1-5}$—alkyl)$_2$ or —O—$C_{1-5}$—alkyl, p cannot be less than or equal to 7 ; or

(a3) m = 1,

A = O atom,

X = OH,

Y = —S—$C_{1-5}$—alkyl, —S—amino—$C_{2-5}$—alkyl, S—carboxy—$C_{1-5}$—alkyl, Z = O atom ; or

(b) m = 1,

A = S atom,

X = Y = OH,

Z = O atom ; or

(c) m = 0,

X = Y = OH, —O—$C_{1-5}$—alkyl, —O—$C_{1-5}$—alkylene—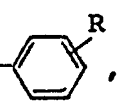, —O—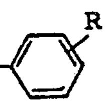, —O—Si ($C_{1-5}$—alkyl)$_3$,

R = OH, $C_{1-5}$—alkyl, —O—$C_{1-5}$—alkyl or is absent,

Z = O atom,

and derivatives thereof, in which one of the two 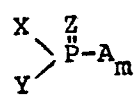 groups has been replaced by a $C_{1-5}$—alkoxy group,

and the alkali metal derivatives and salts with ammonia and amines of the acids of phosphorus and of carboxylic acids,

(a1) with

m = 1,

A = O atom,

X = Cl or OH and Y = X or —O—$C_{1-5}$—alkyl,

Z = O or S atom ;

but where X = OH, Y = —O—$C_{1-5}$—alkyl and Z = O atom, p cannot be less than or equal to 100, a polyalkylene glycol is reacted with phosphorus oxychloride or pyrophosphoryl chloride with or without a solvent in the presence of an acid-binding agent, the phosphoric acid halides obtained are optionally hydrolyzed with water or first with half the stoichiometric quantity of a $C_{1-5}$ alcanol then with water to form the free phosphoric acid ; or

a polyalkylene glycol is melted with a mixture of phosphorous pentoxide and 85 % phosphoric acid (condensed phosphoric acids) ; or

a polyalkylene glycol is reacted with thiophosphoryl chloride in a solvent, saponified at a pH of approximately 7 in the presence of lithium hydroxide and the free acid is liberated in the usual manner ;

(a2) with

A = O atom,

X = —O—$C_{1-5}$—alkyl, —O—$C_{1-5}$—alkylene—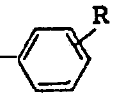, —O—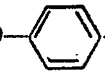—$NO_2$, —O—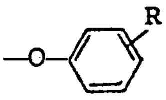,

—NH—$C_{1-5}$—alkyl, —N($C_{1-5}$—alkyl)$_2$, —NH—$C_{1-5}$—alkylene—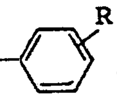, —N($C_{1-5}$—alkylene—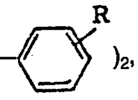)$_2$,

—NH—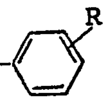, —N(—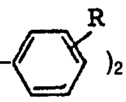)$_2$

Y = X or —O—$C_{1-5}$—alkyl,
R = OH, $C_{1-5}$—alkyl, —O—$C_{1-5}$—alkyl or is absent,
Z = O atom ;
but where X = —N($C_{1-5}$—alkyl)$_2$ or —O—$C_{1-5}$—alkyl, p cannot be less than or equal to 7, the free phosphoric acids cbtained according to (a1) are esterified in the usual manner or the phosphoric acid halides obtained according to (a1) are reacted directly with alcohols or amines ;

(a3) with
A = O atom,
X = OH,
Y = —S—$C_{1-5}$—alkyl, —S—amino—$C_{2-5}$—alkyl, S—carboxy—$C_{1-5}$—alkyl,
Z = O atom ;
a polyalkylene glycol is reacted with thiophosphoryl chloride in a solvent, saponified at a pH of approximately 7 in the presence of lithium hydroxide, the compounds obtained having terminal $(LiO)_2P(S)$—O— radicals are reacted with a trialkylphosphate in an aqueous medium, a aminoalkyl halide having 2 to 5 carbon atoms or with its hydrohalide or with a halocarboxylic acid having 1 to 5 carbon atoms in the alkyl chain ;

(b) with
A = S atom,
X = Y = CH,
Z = O atom ;
a polyalkylene glycol is reacted with a thionyl halide in a solvent in the presence of an amine, the resulting dihalide having terminal halogen atoms is reacted in a Åkerfeldt reaction to form compounds having $(O^-)_2P(O)$—S— radicals and optionally the free acid is obtained in known manner ;

(c) with

X = Y = OH, —O—$C_{1-5}$—alkyl, —O—$C_{1-5}$—alkylene—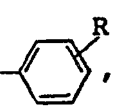, —O—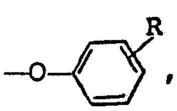, —O—Si($C_{1-5}$—alkyl)$_3$,

R = OH, $C_{1-5}$—alkyl, —O—$C_{1-5}$—alkyl or is absent,
Z = O atom,
a polyether obtained according to (b) having terminal halogen atoms is subjected to a Michaelis-Arbusov reaction and reacted with an optionally nuclear-substituted triphenyl phosphite or a trialkyl phosphite to phosphonic acid esters or a polyether with terminal halogen atoms is reacted in a Michaelis-Becker reaction with alkali phosphorous acid dialkyl esters in a solvent and the obtained esters are optionally saponified directly with acids to form the free phosphonic acids or the phosphonic acid esters are first reacted with a trialkylsilyl halide and then saponified with water,
wherein for the manufacture of compounds of the general formula in which compounds one of the two YXP(Z)—O—groups is replaced by an alkoxy group having 1 to 5 carbon atoms a polyalkylene glycol in which one of the terminal OH radicals is etherified by a $C_{1-5}$ alkyl group is used as starting material in the process according to (a1), (a2), (a3), (b) or (c), and
wherein for the manufacturing of compounds of the general formula in form of salts, the acids of the general formula obtained according to (a1), (a2), (a3), (b) or (c) are neutralized as usual.

2. Process according to claim 1, characterised in that in the general formula according to claim 1, $C_{2-3}$—alkylene = —$CH_2$—$CH_2$—, —$CH_2$—$CH_2$—$CH_2$—, —$CH_2$—CH($CH_3$)— or mixtures thereof ; p = from 3 to 250 and preferably from 30 to 160.

3. Process according to claim 1 or 2, characterised in that in the general formula according to claim 1, —O—$C_{1-5}$—alkyl = methoxy, ethoxy or propoxy, —O—$C_{1-5}$—alkylene—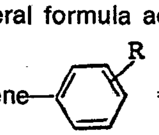 = —O—$C_1$—

alkylene—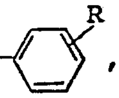, —NH—$C_{1\text{-}5}$—alkyl = methylamino, ethylamino or propylamino, —N($C_{1\text{-}5}$—alkyl)$_2$ = dimethylamino, diethylamino or dipropylamino, —NH—$C_{1\text{-}5}$—alkylene—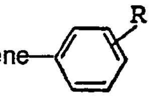 = —NH—$C_1$—alkylene—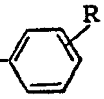, —N($C_{1\text{-}5}$—alkylene—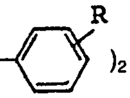)$_2$ = —N($C_1$—alkylene—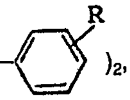)$_2$, —S—$C_{1\text{-}5}$—alkyl = —S—$C_{1\text{-}3}$—alkyl, —S—amino—$C_{2\text{-}5}$—alkyl = —S—amino—$C_{2\text{-}3}$—alkyl, —S—carboxy—$C_{1\text{-}5}$—alkyl = —S—carboxy—$C_{1\text{-}3}$—alkyl and/or —O—Si($C_{1\text{-}5}$—alkyl)$_3$ = —O—Si($CH_3$)$_3$ for X and/or Y.

4. Process according to any one of the preceding claims, characterised in that in the general formula according to claim 1, $C_{1\text{-}5}$—alkyl = methyl, ethyl or propyl and/or —O—$C_{1\text{-}5}$—alkyl = methoxy, ethoxy or propoxy for R.

5. Process according to any one of the preceding claims, characterised in that acids of the general formula according to claim 1 are neutralised to ammonium, Li, Na or K salts.

**Revendications** (pour les Etats contractants : CH, DE, FR, GB, LI, LU, NL, SE)

1. Composés répondant à la formule générale

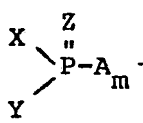 —$C_{2\text{-}3}$—alkylène—O—($C_{2\text{-}3}$—alkylène—O)$_p$—$C_{2\text{-}3}$—alkylène 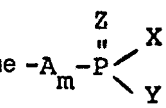

dans laquelle p représente un nombre de 1 à 800 et

(a1) m est égal à 1,

A représente un atome d'oxygène,

X représente Cl ou OH,

Y a la même signification que X ou représente un radical alcoxy contenant de 1 à 5 atomes de carbone,

Z représente un atome d'oxygène ou de soufre, avec la restriction que p ne peut être inférieur ou égal à 100 lorsque X est un OH, Y un alcoxy en $C_1$-$C_5$ et Z un atome d'oxygène, ou

(a2) m est égal à 1,

A représente un atome d'oxygène,

X représente un alcoxy en $C_1$-$C_5$ ou un radical —O—$C_{1\text{-}5}$—alkylène—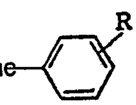, —O—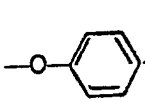—$NO_2$, —O—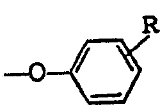, —NH—$C_{1\text{-}5}$—alkyle, —N($C_{1\text{-}5}$—alkyl)$_2$, —NH—$C_{1\text{-}5}$—alkylène—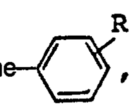, —N($C_{1\text{-}5}$—alkylène—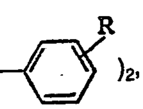)$_2$, —NH—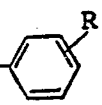 ou —N(—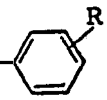)$_2$.

Y a la même signification que X ou représente un alcoxy en $C_1$-$C_5$,

R représente OH, un alkyle en $C_1$-$C_5$, un alcoxy en $C_1$-$C_5$, ou fait défaut, et

Z représente un atome d'oxygène, avec la restriction que p ne peut être inférieur ou égal à 7 lorsque X représente un radical —N($C_{1\text{-}5}$—alkyl)$_2$ ou un alcoxy en $C_1$-$C_5$, ou

(a3) m est égal à 1,

A représente un atome d'oxygène,

X représente OH,

Y représente un alkylthio en $C_1$-$C_5$, un amino—$C_{2\text{-}5}$—alkylthio ou un carboxy—$C_{1\text{-}5}$—alkylthio, et,

Z représente un atome d'oxygène, ou

(b) m est égal à 1,

A représente un atome de soufre,
X et Y représentent chacun un radical OH et
Z représente un atome d'oxygène, ou
(c) m est égal à 0,
X et Y représentent chacun un OH, un alcoxy en $C_1$-$C_5$ ou un radical —O—$C_{1-5}$—alkylène 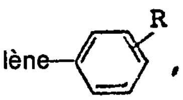, —O— 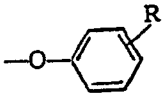 ou —O—Si($C_{1-5}$—alkyl)$_3$,

R représente un OH, un alkyle en $C_1$-$C_5$ ou un alcoxy en $C_1$-$C_5$ ou fait défaut, et
Z représente un atome d'oxygène,

ainsi que leurs dérivés dans lesquels l'un des deux radicaux 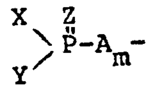

est remplacé par un radical alcoxy contenant de 1 à 5 atomes de carbone, et les dérivés de métaux alcalins et les sels avec l'ammoniac et des amines des acides dérivant du phosphore et des acides carboxyliques.

2. Composés selon la revendication 1 caractérisés en ce que, dans la formule générale de cette revendication 1, le radical $C_{2-3}$—alkylène est un radical —$CH_2$—$CH_2$—, —$CH_2$—$CH_2$—$CH_2$—, —$CH_2$—$CH(CH_3)$— ou un mélange de ces radicaux, et p est un nombre de 3 à 250, de préférence de 30 à 160.

3. Composés selon l'une des revendications 1 et 2, caractérisés en ce que, pour les symboles X et/ou Y présents dans la formule générale de la revendication 1 :
le radical —O—$C_{1-5}$—alkyl est un radical méthoxy, éthoxy ou propoxy,

le radical —O—$C_{1-5}$—alkylène 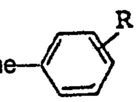 est un radical —O—$C_1$—alkylène 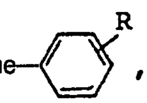,

le radical —NH—$C_{1-5}$—alkyl est un radical méthylamino, éthylamino ou propylamino,
le radical —N($C_{1-5}$—alkyl)$_2$ est un radical diméthylamino, diéthylamino ou dipropylamino,

le radical —NH—$C_{1-5}$—alkylène 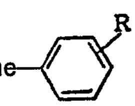 est un radical —NH—$C_1$—alkylène 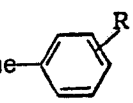,

le radical —N($C_{1-5}$—alkylène 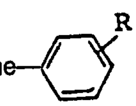 )$_2$ est un radical —N($C_1$—alkylène 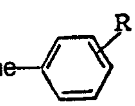 )$_2$,

le radical alkylthio en $C_1$-$C_5$ est un alkylthio en $C_1$-$C_3$,
le radical amino —$C_{2-5}$—alkylthio est un amino —$C_{2-3}$—alkylthio,
le radical carboxy —$C_{1-5}$—alkylthio est un carboxy —$C_{1-3}$—allkylthio et/ou
le radical O—Si($C_{1-5}$—alkyl)$_3$ est un radical —O—Si($CH_3$)$_3$.

4. Composés selon l'une quelconque des revendications précédentes, caractérisés en ce que, pour le symbole R dans la formule générale de la revendication 1 :
le radical —$C_{1-5}$—alkyl est un radical méthyle, éthyle ou propyle et/ou
le radical —O—$C_{1-5}$—alkyl est un radical méthoxy, éthoxy ou propoxy.

5. Composés selon l'une quelconque des revendications précédentes, caractérisés en ce qu'ils sont sous la forme de leurs sels d'ammonium, de lithium, de sodium ou de potassium, de préférence des acides du phosphore.

**Revendications** (pour l'Etat contractant AT)

1. Procédés pour préparer des bis-(mono-esters phosphoriques) de poly-alkylène-glycols, des acides poly-alkylène-glycol-bis-phosphoriques et des dérivés de ces composés, procédé caractérisé en ce que, pour préparer des composés répondant à la formule générale

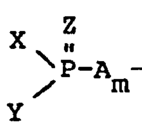 —$C_{2-3}$—alkylène—O—($C_{2-3}$—alkylène—O)$_p$—$C_{2-3}$—alkylène 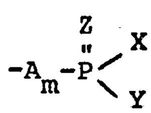

dans laquelle

p représente un nombre de 1 à 800 et

(a1) m est égal à 1,

A représente un atome d'oxygène,

X représente Cl ou OH,

Y a la même signification que X ou représente un radical alcoxy contenant de 1 à 5 atomes de carbone,

Z représente un atome d'oxygène ou de soufre, avec la restriction que p ne peut être inférieur ou égal à 100 lorsque X est un OH, Y un alcoxy en $C_1$-$C_5$ et Z un atome d'oxygène, ou

(a2) m est égal à 1,

A représente un atome d'oxygène,

X représente un alcoxy en $C_1$-$C_5$ ou un radical —O—$C_{1-5}$—alkylène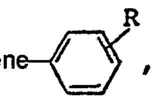,

—O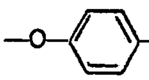—$NO_2$, —O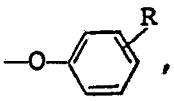, —NH—$C_{1-5}$—alkyle, —N($C_{1-5}$—alkyl$)_2$, —NH—$C_{1-5}$—alkylène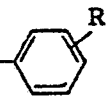, —N($C_{1-5}$—alkylène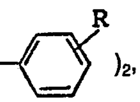$)_2$, —NH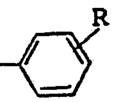 ou —N(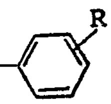$)_2$,

Y a la même signification que X ou représente un alcoxy en $C_1$-$C_5$,

R représente un OH, un alkyle en $C_1$-$C_5$, un alcoxy en $C_1$-$C_5$, ou fait défaut, et

Z représente un atome d'oxygène, avec la restriction que p ne peut être inférieur ou égal à 7 lorsque X représente un radical —N($C_{1-5}$—alkyl$)_2$ ou un alcoxy en $C_1$-$C_5$, ou

(a3) m est égal à 1,

A représente un atome d'oxygène,

X représente un OH,

Y représente un alkylthio en $C_1$-$C_5$, un amino —$C_{2-5}$—alkylthio ou un carboxy —$C_{1-5}$—alkylthio, et

Z représente un atome d'oxygène, ou

(b) m est égal à 1,

A représente un atome de soufre,

X et Y représentent chacun un radical OH et

Z représente un atome d'oxygène, ou

(c) m est égal à 0,

X et Y représentent chacun un OH, un alcoxy en $C_1$-$C_5$ ou un radical —O—$C_{1-5}$—alkylène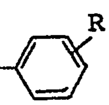, —O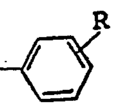 ou —O—Si($C_{1-5}$—alkyl$)_3$,

R représente un OH, un alkyle en $C_1$-$C_5$ ou un alcoxy en $C_1$-$C_5$ ou fait défaut, et

Z représente un atome d'oxygène,

ainsi que leurs dérivés dans lesquels l'un des deux radicaux 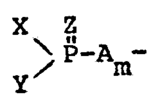 est remplacé par un radical alcoxy contenant de 1 à 5 atomes de carbone, et

leurs dérivés des métaux alcalins et les sels avec l'ammoniac et des amines des acides dérivant du phosphore et des acides carboxyliques,

procédé caractérisé en ce que :

(a1) dans le cas où :

m est égal à 1,

A représente un atome d'oxygène,

X représente Cl ou OH,

Y a la même signification que X ou représente un radical alcoxy contenant de 1 à 5 atomes de carbone,

Z représente un atome d'oxygène ou de soufre,

avec la restriction que p ne peut être inférieur ou égal à 100 lorsque X est un OH, Y un alcoxy en $C_1$-$C_5$ et Z un atome d'oxygène,

on fait réagir un poly-alkylène-glycol avec l'oxychlorure de phosphore ou le chlorure de pyrophosphoryle, en utilisant ou non un solvant, en présence d'un accepteur d'acides, et on hydrolyse éventuellement les halogénures d'acides phosphoriques obtenus, avec de l'eau ou d'abord avec la moitié de la quantité stoechiométrique d'un alcanol en $C_1$-$C_5$, puis avec de l'eau, pour obtenir l'acide phosphorique libre ; ou on fait fondre un poly-alkylène-glycol avec un mélange d'anhydride phosphorique et d'acide phosphorique à 85 % (acides phosphoriques condensés) ; ou on fait réagir un polyalkylène-glycol avec le chlorure de phosphorothioyle dans un solvant, on saponifie à un pH d'environ 7 en présence d'hydroxyde de lithium et on libère l'acide de la manière habituelle ;

(a2) dans le cas où :

m est égal à 1,

A représente un atome d'oxygène,

X représente un alcoxy en $C_1$-$C_5$ ou un radical —O—$C_{1\text{-}5}$—alkylène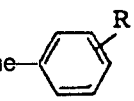,

—O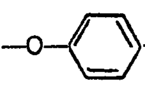—$NO_2$, —O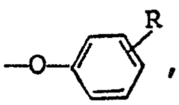, —NH—$C_{1\text{-}5}$—alkyl, —N($C_{1\text{-}5}$—alkyl)$_2$, —NH—$C_{1\text{-}5}$—alkylène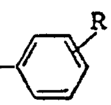,

—N($C_{1\text{-}5}$—alkylène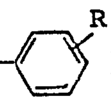)$_2$, —NH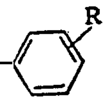 ou —N(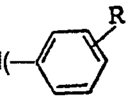)$_2$,

Y a la même signification que X ou représente un alcoxy en $C_1$-$C_5$,

R représente OH, un alkyle en $C_1$-$C_5$, un alcoxy en $C_1$-$C_5$, ou fait défaut, et

Z représente un atome d'oxygène,

avec la restriction que p ne peut être inférieur ou égal à 7 lorsque X représente un radical —N($C_{1\text{-}5}$—alkyl)$_2$ ou un alcoxy en $C_1$-$C_5$,

on estérifie de la manière habituelle les acides phosphoriques libres obtenus selon (a1) ou on fait réagir directement avec des alcools ou des amines les halogénures d'acides phosphoriques obtenus selon (a1) ;

(a3) dans le cas où :

m est égal à 1,

A représente un atome d'oxygène,

X représente OH,

Y représente un alkylthio en $C_1$-$C_5$, un amino —$C_{2\text{-}5}$—alkylthio ou un carboxy —$C_{1\text{-}5}$—alkylthio, et,

Z représente un atome d'oxygène,

on fait réagir un poly-alkylène-glycol avec le chlorure de phosphorothioyle dans un solvant, on saponifie à un pH d'environ 7 en présence d'hydroxyde de lithium, et on fait réagir les composés obtenus, qui contiennent des radicaux $(LiO)_2P(S)$—O— terminaux, avec un phosphate de trialkyle en milieu aqueux, avec un halogénure d'amino-alkyle contenant de 2 à 5 atomes de carbone ou son halohydrate ou avec un acide halogéno-carboxylique contenant, dans sa chaîne alkyle, de 1 à 5 atomes de carbone ;

(b) dans le cas où :

m est égal à 1,

A représente un atome de soufre,

X et Y représentent chacun un radical OH et

Z représente un atome d'oxygène,

on fait réagir un poly-alkylène-glycol avec un halogénure de thionyle dans un solvant en présence d'une amine, on transforme le dihalogénure obtenu, qui contient des atomes d'halogènes terminaux, par une réaction d'Åkerfeldt en composés contenant des radicaux $(O^-)_2P(O)$—S— et éventuellement on obtient l'acide libre de manière connue ;

(c) dans le cas où :

m est égal à 0,

X et Y représentent chacun un OH ou un radical —O—$C_{1\text{-}5}$—alkyl, —O—$C_{1\text{-}5}$—alkylène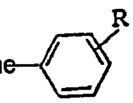, —O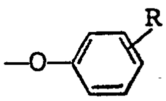 ou —O—Si($C_{1\text{-}5}$—alkyl)$_3$,

R représente un OH, un alkyle en $C_1$-$C_5$ ou un alcoxy en $C_1$-$C_5$, ou fait défaut, et

Z représente un atome d'oxygène,

on soumet à une réaction de Michaelis-Arbusov un polyéther à atomes d'halogènes terminaux qui a été obtenu selon (b) et on fait réagir le composé obtenu avec un phosphite de triphényle éventuellement porteur de substituants sur ses noyaux ou avec un phosphite de trialkyle pour obtenir des esters phosphoniques, ou on effectue une réaction de Michaelis-Becker sur un polyéther à atomes d'halogènes

terminaux avec des sels de métaux alcalins de phosphites de dialkyles dans un solvant et on saponifie éventuellement les esters obtenus, directement avec des acides, pour les convertir en acides phosphoniques libres ou on fait réagir les esters phosphoniques d'abord avec un halogénure de trialkylsilyle, puis on saponifie par l'eau,

à quoi il convient d'ajouter que :

pour préparer des composés qui répondent à la formule générale et dans lesquels l'un des deux radicaux YXP(Z)—O— est remplacé par un radical alcoxy en $C_1$-$C_5$ on part, dans le procédé selon (a1), (a2), (a3), (b) ou (c), d'un polyalkylène-glycol dont l'un des radicaux OH terminaux est éthérifié par un radical alkyle en $C_1$-$C_5$, et pour préparer des composés répondant à la formule générale sous la forme de sels on neutralise de la manière habituelle les acides de la formule générale qui ont été obtenus selon (a1), (a2), (a3), (b) ou (c).

2. Procédé selon la revendication 1 caractérisé en ce que, dans la formule générale de cette revendication 1, le radical $C_{2\text{-}3}$—alkylène est un radical $—CH_2—CH_2—$, $—CH_2—CH_2—CH_2—$, $—CH_2—CH(CH_3)—$ ou un mélange de ces radicaux, et p est un nombre de 3 à 250, de préférence de 30 à 160.

3. Procédé selon l'une des revendications 1 et 2, caractérisé en ce que, pour les symboles X et/ou Y présents dans la formule générale de la revendication 1 :

le radical —O—$C_{1\text{-}5}$—alkyl est un radical méthoxy, éthoxy ou propoxy,

le radical —O—$C_{1\text{-}5}$—alkylène— 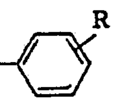 est un radical —O—$C_1$—alkylène— 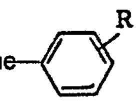

le radical —NH—$C_{1\text{-}5}$—alkyl est un radical méthylamino, éthylamino ou propylamino,

le radical —N($C_{1\text{-}5}$—alkyl)$_2$ est un radical diméthylamino, diéthylamino ou dipropylamino,

le radical —NH—$C_{1\text{-}5}$—alkylène— 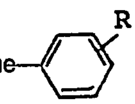 est un radical —NH—$C_1$—alkylène— 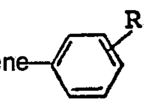,

le radical —NH($C_{1\text{-}5}$—alkylène— 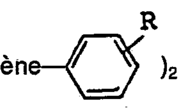 )$_2$ est un radical —NH($C_1$—alkylène— 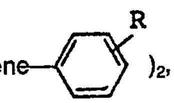 )$_2$,

le radical alkylthio en $C_1$-$C_5$ est un alkylthio en $C_1$-$C_3$,

le radical amino —$C_{2\text{-}5}$—alkylthio est un amino —$C_{2\text{-}3}$—alkylthio,

le radical carboxy —$C_{1\text{-}5}$—alkylthio est un carboxy —$C_{1\text{-}3}$—alkylthio et/ou

le radical —O—Si($C_{1\text{-}5}$—alkyl)$_3$ est un radical —O—Si($CH_3$)$_3$.

4. Procédé selon l'une quelconque des revendications précédentes, caractérisé en ce que, pour le symbole R dans la formule générale de la revendication 1 :

le radical —$C_{1\text{-}5}$—alkyl est un radical méthyle, éthyle ou propyle et/ou

le radical —O—$C_{1\text{-}5}$—alkyl est un radical méthoxy, éthoxy ou propoxy.

5. Procédé selon l'une quelconque des revendications précédentes, caractérisé en ce qu'on neutralise des acides répondant à la formule générale selon la revendication 1 pour les convertir en sels d'ammonium, de lithium, de sodium ou de potassium.